(19) **Europäisches Patentamt European Patent Office Office européen des brevets**

(11) **EP 3 210 583 B1**

(12) **EUROPEAN PATENT SPECIFICATION**

(45) Date of publication and mention
of the grant of the patent:
**11.07.2018 Bulletin 2018/28**

(51) Int Cl.:
*A61F 13/15* (2006.01)     *A61F 13/49* (2006.01)
*A61F 13/53* (2006.01)     *D06M 17/00* (2006.01)
*D06M 17/06* (2006.01)

(21) Application number: **17166338.8**

(22) Date of filing: **13.12.2012**

(54) **WATER-ABSORBENT SHEET COMPOSITE**

WASSERABSORBIERENDER FOLIENVERBUNDSTOFF

COMPOSITE DE FEUILLE ABSORBANT L'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.12.2011 JP 2011285642**

(43) Date of publication of application:
**30.08.2017 Bulletin 2017/35**

(62) Document number(s) of the earlier application(s) in
accordance with Art. 76 EPC:
**12863691.7 / 2 799 047**

(73) Proprietor: **Sumitomo Seika Chemicals Co., Ltd.
Kako-gun, Hyogo 675-0145 (JP)**

(72) Inventors:
• **MATSUSHITA, Hideki
  Hyogo, 672-8076 (JP)**
• **TAKATORI, Junichi
  Hyogo, 672-8076 (JP)**
• **MARUO, Junichi
  Hyogo, 672-8076 (JP)**
• **KUDO, Kana
  Hyogo, 672-8076 (JP)**

(74) Representative: **J A Kemp
14 South Square
Gray's Inn
London WC1R 5JJ (GB)**

(56) References cited:
**EP-A1- 2 301 499      EP-A1- 2 383 115
EP-A1- 2 387 981      EP-A1- 2 524 676
EP-A1- 2 524 679      EP-A1- 2 524 680
WO-A1-2011/136087**

EP 3 210 583 B1

**Description**

<u>TECHNICAL FIELD</u>

The present invention relates to a water-absorbent sheet structure which can be used in the fields of hygienic materials, agricultural fields, fields of construction materials, and the like. More specifically, the present invention relates to a water-absorbent sheet structure which is thin and can be suitably used in absorbent articles, such as light incontinence pads. Furthermore, the present invention relates to an absorbent article such as light incontinence pads using the water-absorbent sheet structure.

<u>BACKGROUND ART</u>

Absorbent articles represented by light incontinence pads or the like have a structure in which an absorbent material for absorbing a liquid such as a body liquid is sandwiched with a flexible liquid-permeable surface sheet (top sheet) positioned on a side contacting a body and a liquid-impermeable backside sheet (back sheet) positioned on a side opposite to that contacting the body.

Conventionally, there have been increasing demands for thinning and light-weighing of absorbent articles, from the viewpoint of designing property, body fittability and convenience upon carrying. Further, in the recent years, there have been growing needs for so-called eco-friendly intentions, in which resources are effectively utilized so that use of natural materials that require a long time to grow such as trees is avoided as much as possible, from the viewpoint of environmental protection.

In view of the above, as a water-absorbent sheet structure having a very small content of crushed pulp fibers and the like of wooden materials, having excellent fundamental properties (fast liquid permeation rate, sufficient liquid absorbent capacity, small amount of liquid re-wet, small liquid leakage amount, and shape retaining ability), and being capable of accomplishing thinning, a water-absorbent sheet structure in which a given amount of a water-absorbent resin and a given amount of hot melt adhesive are sandwiched with two or more hydrophilic nonwoven fabrics having a given basis weight (see, for example, Patent Publications 1 and 2) has been proposed.

EP-A1-2,383,115 discloses a water-absorbent sheet structure comprising a structure in which an absorbent layer comprising a water-absorbent resin and an adhesive is sandwiched with nonwoven fabrics from an upper side and a lower side of the absorbent layer.

<u>PRIOR ART PUBLICATIONS</u>

<u>PATENT PUBLICATIONS</u>

    Patent Publication 1: WO 2010/004894
    Patent Publication 2: WO 2010/004895

<u>SUMMARY OF THE INVENTION</u>

<u>PROBLEMS TO BE SOLVED BY THE INVENTION</u>

The water-absorbent sheet structure disclosed in Patent Publication 1 or 2 has sufficiently excellent fundamental properties mentioned above, and a water-absorbent sheet structure with even more improved properties has been continuously in demand. Particularly when a water-absorbent sheet structure is used for an absorbent article such as light incontinence pads, there are some strong needs for dry feel, while avoiding a wet feel upon use, so that the above-mentioned water-absorbent sheet structure having further improved properties in fast liquid permeation rate, a small amount of liquid re-wet, and a small liquid leakage amount is in demand.

An object of the present invention is to provide a water-absorbent sheet structure that is thin and has excellent absorbent capacities such as liquid permeability and a small amount of liquid re-wet, and an absorbent article using the structure.

<u>MEANS TO SOLVE THE PROBLEMS</u>

The present invention relates to:
a water-absorbent sheet structure comprising a structure in which an absorbent layer comprising a water-absorbent resin and an adhesive is sandwiched with nonwoven fabrics from an upper side and a lower side of the absorbent layer, the water-absorbent sheet structure comprising: an upper nonwoven fabric which is a water-permeable nonwoven fabric having a water permeation rate of 10 s or less; and a lower nonwoven fabric which is a water-retaining nonwoven fabric having an amount of water absorption of 250 g/m$^2$ or more, wherein the absorbent layer is fractionated into a primary absorbent layer and a secondary absorbent layer, with a breathable fractionating layer.

EFFECTS OF THE INVENTION

The water-absorbent sheet structure according to the present invention is thin and has some excellent effects that the sheet structure can sufficiently exhibit absorbent capacities such as fast liquid permeability, a small amount of liquid re-wet, and a small liquid leakage amount. Therefore, the water-absorbent sheet structure according to the present invention is used for an absorbent material for light incontinence pads or the like, whereby hygienic materials which are thin and have excellent body fittability can be provided.

BRIEF DESCRIPTION OF THE DRAWINGS

[Figure 1] A cross-sectional schematic view of a water-absorbent sheet structure not in accordance with the present invention.
[Figure 2] A cross-sectional schematic view of a water-absorbent sheet structure not in accordance with the present invention.
[Figure 3] A cross-sectional schematic view of an embodiment of a water-absorbent sheet structure of the present invention.
[Figure 4] A schematic view showing an outline of the constitution of an equipment for measuring a water-permeation rate of a nonwoven fabric.
[Figure 5] A schematic view showing an outline of the constitution of a strike-through plate, which is a part of an equipment for measuring a water-permeation rate of a nonwoven fabric.
[Figure 6] A schematic view showing an outline of the constitution of an apparatus used for carrying out a slope leakage test for a water-absorbent sheet structure.

MODES FOR CARRYING OUT THE INVENTION

A feature of the water-absorbent sheet structure of the present invention is in that a water-absorbent sheet structure comprises a structure in which an absorbent layer containing a water-absorbent resin and an adhesive is sandwiched with nonwoven fabrics from an upper side and a lower side of the absorbent layer, wherein the water-absorbent structure comprises an upper nonwoven fabric which is a water-permeable nonwoven fabric having excellent water permeability, and a lower nonwoven fabric which is a water-retaining nonwoven fabric having excellent water-retaining property. By taking the above structure, a water-absorbent sheet structure which is thin and has excellent liquid absorbent properties such as fast liquid permeability, a small amount of liquid re-wet, and a small liquid leakage amount can be realized.

In the water-absorbent sheet structure of the present invention, a water-permeable nonwoven fabric having excellent water permeability is used as an upper nonwoven fabric. This is because a liquid to be absorbed inhibits stagnation on the surface of the water-absorbent sheet structure, and is migrated quickly to an absorbent layer containing a water-absorbent resin, thereby inhibiting a wet feel on the surface, and realizing a dry feel. The upper side of an absorbent layer as used herein refers to a side to which a liquid to be absorbed is supplied at the time of preparing an absorbent article using the water-absorbent sheet structure obtained, and the lower side of an absorbent layer refers to a side opposite thereof.

The water-permeation rate of the water-permeable nonwoven fabric in the present invention is 10 s or less, and preferably from 5 to 9 s, from the viewpoint of speeding up a liquid permeation rate of the water-absorbent sheet structure, and inhibiting stagnation of the liquid on the surface of the water-absorbent sheet structure. The water-permeation rate of the nonwoven fabric is a value obtainable by a measurement method described in Examples set forth below.

The water-permeable nonwoven fabrics usable in the present invention are not particularly limited, as long as the water-permeable nonwoven fabrics satisfy the water-permeation rate mentioned above. The nonwoven fabrics include nonwoven fabrics made of fibers selected from the group consisting of synthetic fibers such as polyolefin fibers such as polyethylene (PE) and polypropylene (PP); polyester fibers such as polyethylene terephthalate (PET), polytrimethylene terephthalate (PTT), and polyethylene naphthalate (PEN), and blends thereof, from the viewpoint of liquid permeability, flexibility and strength upon forming into a water-absorbent sheet structure. The nonwoven fabrics made of polyolefin fibers or polyester fibers are even more preferable. In addition, it is preferable that the nonwoven fabrics are spunbond nonwoven fabrics or air-through nonwoven fabrics, from the viewpoint of speeding up the water-permeation rate. Further, nonwoven fabrics made of fibers produced by hydrophilically treating hydrophobic synthetic fibers such as polyolefin fibers and polyester fibers according to a known method are more preferred, from the viewpoint of even more increasing liquid permeability of the water-absorbent sheet structure.

On the other hand, in the water-absorbent sheet structure of the present invention, a water-retaining nonwoven fabric having excellent water-retention property is used as a lower nonwoven fabric. By using a water-permeable nonwoven fabric having a high water permeability as a nonwoven fabric on a side to which a liquid to be absorbed is supplied, a so-called liquid leakage is likely to take place where a liquid flows out from the water-absorbent sheet structure before a water-absorbent resin of the absorbent layer absorbs the liquid. In view of the above, a liquid leakage from the water-absorbent sheet structure can be inhibited by using a water-retaining nonwoven fabric having excellent water-retention property as a nonwoven fabric positioned on an opposite side from a side supplying the liquid to be absorbed.

The amount of water absorption of the water-retaining nonwoven fabric in the present invention is 250 g/m$^2$ or more, and preferably from 300 to 750 g/m$^2$, from viewpoint of inhibiting liquid leakage from the water-absorbent sheet structure. The amount of water absorption of the nonwoven fabric is a value obtainable by a measurement method described in Examples set forth below.

The water-retaining nonwoven fabric in the present invention is not particularly limited, as long as the nonwoven fabric satisfies the amount of water absorption mentioned above. The water-retaining nonwoven fabric is preferably nonwoven fabrics made of polyamide fibers such as nylon, rayon fibers, acetate fibers, and other synthetic fibers, nonwoven fabrics made of blends of cotton, silk, hemp, pulp (cellulose) fibers, or the like. More specifically, spunbond nonwoven fabrics made of polyamide fibers such as nylon and spunlace nonwoven fabrics made of rayon fibers as a main component are more preferred. As the above-mentioned spunlace nonwoven fabrics, those made of rayon fibers, as a main component, properly blended with polyolefin fibers and/or polyester fibers are preferably used, among which rayon/PET nonwoven fabrics and rayon/PP/PE nonwoven fabrics are preferably used. The above-mentioned nonwoven fabrics may contain a small amount of pulp fibers to an extent that would not increase a thickness of the water-absorbent sheet structure.

The water-absorbent sheet structure of the present invention may take an embodiment where hydrophilic fibers such as pulp fibers may be admixed together with a water-absorbent resin in the absorbent layer between the nonwoven fabrics in an amount that would not hamper the effects of the present invention, and an embodiment of not substantially including hydrophilic fibers is preferred, from the viewpoint of thinning.

As the kinds of the water-absorbent resins in the water-absorbent sheet structure of the present invention, commercially available water-absorbent resins can be used. For example, the water-absorbent resin includes hydrolysates of starch-acrylonitrile graft copolymers, neutralized products of starch-acrylic acid graft polymers, saponified products of vinyl acetate-acrylic acid ester copolymers, crosslinked products of partially neutralized polymer of acrylic acid, partially neutralized products of polyacrylic acid, and the like. Among them, the crosslinked products of partially neutralized polymer of acrylic acid are preferred, from the industrial viewpoints such as supplying capacity and costs. Methods for synthesizing crosslinked products of partially neutralized polymer of acrylic acid include reversed phase suspension polymerization method, and aqueous solution polymerization method.

The content of the water-absorbent resin in the water-absorbent sheet structure is preferably from 100 to 1,000 g per one square-meter of the water-absorbent sheet structure, i.e. 100 to 1,000 g/m$^2$, more preferably from 150 to 900 g/m$^2$, and even more preferably from 200 to 700 g/m$^2$, from the viewpoint of obtaining sufficient liquid absorbent properties even when a water-absorbent sheet structure of the present invention is used for an absorbent article. The content is preferably 100 g/m$^2$ or more, from the viewpoint of exhibiting sufficient liquid absorbent properties as a water-absorbent sheet structure, thereby inhibiting especially an amount of liquid re-wet, and the content is preferably 1,000 g/m$^2$ or less, from the viewpoint of improving a liquid permeation rate.

The adhesive usable in the water-absorbent sheet structure of the present invention includes, for example, rubber-based adhesives such as natural rubbers, butyl rubbers, and polyisoprene; styrene-based elastomer adhesives such as styrene-isoprene-styrene block copolymers (SIS), styrene-butadiene-styrene block copolymers (SBS), styrene-isobutylene-styrene block copolymers (SIBS), and styrene-ethylene-butylene-styrene block copolymers (SEBS); ethylene-vinyl acetate copolymer (EVA) adhesives; ethylene-acrylic acid derivative copolymer-based adhesives such as ethylene-ethyl acrylate copolymers (EEA), and ethylene-butyl acrylate copolymers (EBA); ethylene-acrylic acid copolymer (EAA) adhesives; polyamide-based adhesives such as copolymer nylons and dimer acid-based polyamides; polyolefin-based adhesives such as polyethylenes, polypropylenes, atactic polypropylenes, and copolymeric polyolefins; polyester-based adhesives such as polyethylene terephthalate (PET), polybutylene terephthalate (PBT), and copolymeric polyesters; and acrylic-based adhesives. In the present invention, at least one member selected from the group consisting of the ethylene-vinyl acetate copolymer adhesives, the styrene-based elastomer adhesives, the polyolefin-based adhesives, and the polyester-based adhesives is preferred, from the viewpoint of high adhesive strength, thereby making it possible to prevent exfoliation of a nonwoven fabric and scattering of the water-absorbent resin in the water-absorbent sheet structure. These adhesives may be used alone, or they may be used in combination of two or more kinds.

When a thermal-fusing adhesive is used, the melting temperature or a softening temperature of the adhesive is preferably from 50° to 180°C, and more preferably from 70° to 150°C, from the viewpoint of sufficiently fixing a water-absorbent resin to a nonwoven fabric, and at the same time preventing thermal deterioration or deformation of the nonwoven fabric.

The content of the adhesive in the water-absorbent sheet structure is preferably within the range of from 0.05 to 2.0 times, more preferably within the range of from 0.08 to 1.5 times, and even more preferably within the range of from 0.1 to 1.0 time, based on the content of the water-absorbent resin on a mass basis. The content of the adhesive is preferably 0.05 times or more, from the viewpoint of having sufficient adhesion, thereby preventing exfoliation of the nonwoven fabrics themselves or scattering of the water-absorbent resin, and increasing shape retaining ability of a water-absorbent sheet structure. The content of the adhesive is preferably 2.0 times or less, from the viewpoint of avoiding the inhibition of the swelling of the water-absorbent resin due to too strong adhesion to each other, thereby improving a liquid permeation rate or liquid leakage of a water-absorbent sheet structure.

In the water-absorbent sheet structure of the present invention, the absorbent layer formed between the nonwoven fabrics contains at least a water-absorbent resin and an adhesive, and the water-absorbent sheet structure is formed by, for example, evenly dispersing a mixed powder of a water-absorbent resin and an adhesive on a nonwoven fabric, further overlaying with a nonwoven fabric, and subjecting overlaid layers to heating, if necessary, heating under pressure, near a melting temperature of the adhesive. Alternatively, the water-absorbent sheet structure of the present invention is formed by evenly dispersing a water-absorbent resin over an adhesive-coated nonwoven fabric, further overlaying with an adhesive-coated nonwoven fabric, and subjecting overlaid layers to heating, if necessary, under pressure, or the water-absorbent sheet structure is formed by sandwiching a water-absorbent resin between the nonwoven fabrics, and thereafter subjecting the overlaid layers to thermal embossing or the like.

Specifically, the water-absorbent sheet structure of the present invention can be produced by, for example, a method as described hereinbelow.

(a) A mixed powder of a water-absorbent resin and an adhesive is evenly dispersed over a nonwoven fabric, a nonwoven fabric is further overlaid thereto, and the overlaid layers are subjected to pressing, while heating near a melting temperature of the adhesive.

(b) A mixed powder of a water-absorbent resin and an adhesive is evenly dispersed over a nonwoven fabric, and passed through a heating furnace to fix the powder to an extent that the powder does not scatter. A nonwoven fabric is overlaid thereto, and the overlaid layers are subjected to pressing while heating.

(c) An adhesive is melt-coated over a nonwoven fabric, a water-absorbent resin is immediately thereafter evenly dispersed thereto to form a layer, and further a nonwoven fabric to which an adhesive is melt-coated is overlaid from an upper side in a manner that an adhesive-coated side is facing the side of the dispersed water-absorbent resin layer, and the overlaid layers are subjected to pressing, if necessary, with heating, using a roller press or the like to subject to pressure adhesion.

(d) A mixed powder of a water-absorbent resin and an adhesive is evenly dispersed over a nonwoven fabric, further a nonwoven fabric is overlaid thereto, and the overlaid layers are subjected to thermal embossing to heat-and-pressure adhesion the hydrophilic nonwoven fabrics themselves.

A water-absorbent sheet structure having a structure that an absorbent layer containing a water-absorbent resin is sandwiched with nonwoven fabrics from an upper side and a lower side can be obtained by, for example, producing a water-absorbent sheet structure according to the method shown in any one of these (a) to (d). Among these methods, the methods of (a), (c) and (d) are more preferred, from the viewpoint of convenience in the production method and high production efficiency. A water-absorbent sheet structure can be also produced by combining the methods exemplified in (a) to (d).

In addition, the water-absorbent sheet structure of the present invention may be properly blended with an additive such as deodorant, an anti-bacterial agent, or a gel stabilizer.

In the present invention, the above-mentioned water-absorbent sheet structure has a structure in which a part or entire side of the absorbent layer thereof is fractionated into a primary absorbent layer on a side to which a liquid to be absorbed is supplied and a secondary absorbent layer on a side opposite thereto by using an appropriate breathable fractionating layer in a perpendicular direction (the thickness direction of the sheet structure). By having the above structure, the liquid absorbent properties of the water-absorbent sheet structure, especially liquid leakage property, are improved.

The above-mentioned breathable fractionating layer has appropriate breathability and liquid-permeability, so long as the breathable fractionating layer is a layer in which a particle-form substance such as a water-absorbent resin does not substantially pass therethrough. Specific examples thereof include reticular products such as nets having fine pores made of PE or PP fibers; porous films such as perforated films; sanitary papers such as tissue paper; and cellulose-containing synthetic fiber nonwoven fabrics such as airlaid nonwoven fabrics made of pulp/PE/PP, or nonwoven fabrics made of synthetic fibers, such as rayon fibers, polyolefin fibers, and polyester fibers; and the like.

A mass ratio of the water-absorbent resin of the primary absorbent layer to the water-absorbent resin of the secondary absorbent layer mentioned above, i.e. the primary absorbent layer/the secondary absorbent layer, is preferably from 98/2 to 20/80, more preferably from 95/5 to 25/75, and even more preferably from 90/10 to 30/70. The primary absorbent layer/the secondary absorbent layer is preferably 98/2 or less, from the viewpoint of sufficiently exhibiting liquid absorbent properties of the secondary absorbent layer, thereby inhibiting liquid leakage, and is preferably 20/80 or more, from the viewpoint of increasing liquid permeability and preventing a wet feel on a side contacting the body (skin) after the absorption.

The liquid absorbent properties of the water-absorbent sheet structure of the present invention are influenced by the water-absorbent properties of the water-absorbent resin used. In the water-absorbent resin, it is preferable to select one having suitable water-absorbent properties taking the constitution of each of the components and the like of the water-absorbent sheet structure into consideration. In addition, for example, in a case where an absorbent layer is to have a two-layer structure, the water-absorbent resins of the primary absorbent layer and the secondary absorbent layer may be identical or different.

The water-absorbent sheet structure of the present invention has one of the features in enabling thinning of the water-absorbent sheet structure. Taking its use in absorbent articles into consideration, the thickness of the water-absorbent sheet structure, in a dry state, is preferably 4 mm or less, more preferably 3 mm or less, and even more preferably from 1.0 to 2.5 mm. The dry state refers to a state before which the water-absorbent sheet structure absorbs a liquid. In the present specification, the thickness of the water-absorbent sheet structure in a dry state is a value obtainable by a measurement method described in Examples set forth below.

Also, the water-absorbent sheet structure of the present invention has one of the features in a fast liquid permeation rate. Taking its use in absorbent articles into consideration, a total liquid permeation rate of the water-absorbent sheet structure is preferably 600 s or less, and more preferably 550 s or less. In the present specification, the total liquid permeation rate of the water-absorbent sheet structure is a value obtainable by a measurement method described in Examples set forth below.

Further, the water-absorbent sheet structure of the present invention has one of the features in a small amount of liquid re-wet. Taking its use in absorbent articles into consideration, the amount of liquid re-wet of the water-absorbent sheet structure is preferably 10 g or less, and more preferably 8 g or less. In the present specification, the amount of liquid re-wet of the water-absorbent sheet structure is a value obtainable by a measurement method described in Examples set forth below.

Furthermore, the water-absorbent sheet structure of the present invention has one of the features in a small liquid slope leakage. Taking its use in absorbent articles into consideration, the total slope leakage amount of the water-absorbent sheet structure is preferably 1.0 g or less, and more preferably 0.8 g or less. In the present specification, the total slope leakage amount of the water-absorbent sheet structure is a value obtainable by a measurement method described in Examples set forth below.

As the water-absorbent sheet structure of the present invention, those having given properties for a thickness in a dry state, a total liquid permeation rate, an amount of liquid re-wet, and a total slope leakage amount are preferred.

Further, since the water-absorbent sheet structure of the present invention has a very small amount of a material derived from nature, consideration has been made to the environment while having high performance in a thickness, a liquid permeation rate, and an amount of liquid re-wet as mentioned above. The proportion of the natural material used is preferably 30% by mass or less, more preferably 20% by mass or less, and even more preferably 15% by mass or less. The proportion of the natural material used is calculated by dividing a total content of pulp, cotton, hemp, silk, and the like contained in very small amounts as the constituents of the water-absorbent sheet structure by the mass of the water-absorbent sheet structure.

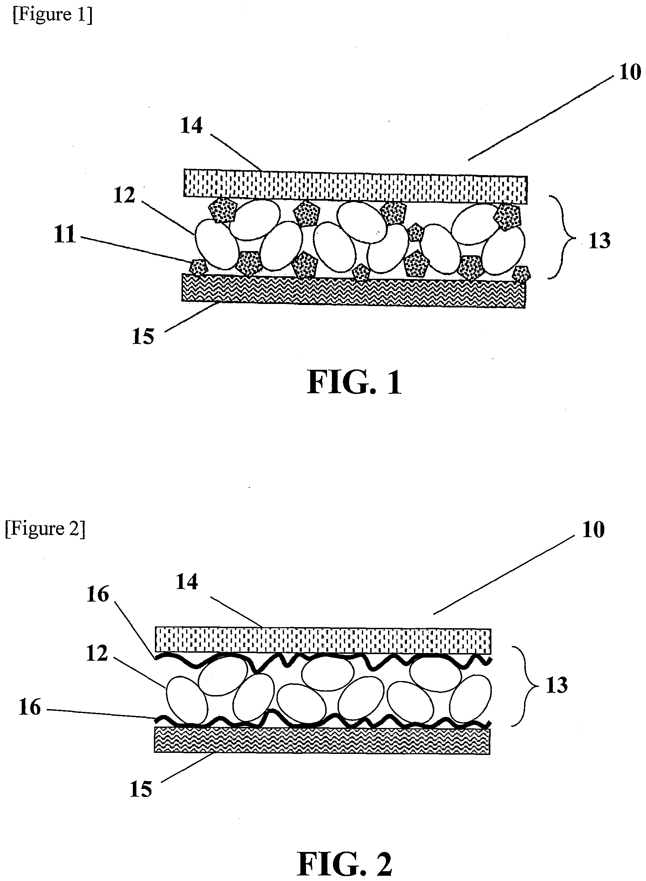

Next, the structure of a water-absorbent sheet structure not in accordance with the present invention will be explained by referring to Figure 1. Here, Figure 1 is an enlarged cross-sectional view schematically showing a structure of a water-absorbent sheet structure.

A water-absorbent sheet structure 10 shown in Figure 1 comprises a structure in which an absorbent layer 13 containing a water-absorbent resin 12 and an adhesive 11 is sandwiched with a water-permeable nonwoven fabric 14 and a water-retaining nonwoven fabric 15 from an upper side and a lower side of the absorbent layer.

In addition, the water-absorbent sheet structure shown in Figure 2 is also an illustration of another water-absorbent sheet structure not in accordance with the present invention. Figure 2 shows an example where an adhesive 16 is coated in a molten state to a water-permeable nonwoven fabric 14 and a water-retaining nonwoven fabric 15.

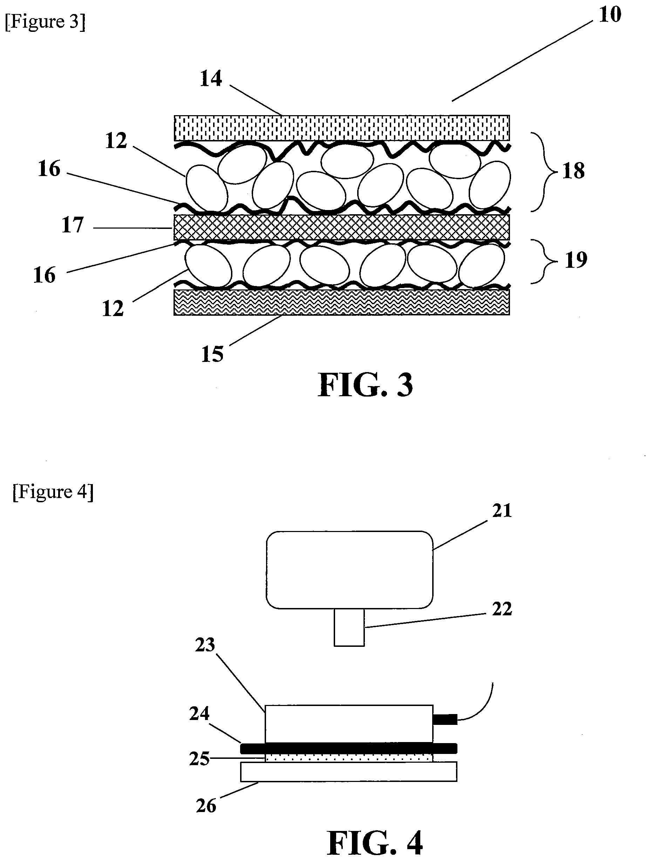

Further, the water-absorbent sheet structure shown in Figure 3 is an illustration of an embodiment of a water-absorbent sheet structure of the present invention. Figure 3 shows a structure where an absorbent layer is fractionated into a primary absorbent layer 18 and a secondary absorbent layer 19, with a breathable fractionating layer 17, and sandwiched with a water-permeable nonwoven fabric 14 and a water-retaining nonwoven fabric 15 from an upper side and a lower side of the absorbent layer.

The absorbent article according to the present invention can be obtained by sandwiching a water-absorbent sheet structure of the present invention between a liquid-permeable sheet and a liquid-impermeable sheet. As the liquid-permeable sheet and the liquid-impermeable sheet mentioned above, known ones in the technical field of the absorbent articles can be used without particular limitations, and an embodiment where a liquid-permeable sheet is placed on an upper side, and a liquid-impermeable sheet is placed in a lower side, respectively, can be used. Also, the absorbent article can be produced by a known method.

The above-mentioned absorbent article includes, for example, disposable diapers, light incontinence pads, sanitary napkins, pet sheets, drip sheets for foods, water blocking materials for electric power cables, and the like.

EXAMPLES

The present invention will be specifically described hereinbelow by the Examples, without intending to limit the scope of the present invention thereto.

The properties of the nonwoven fabric were measured in accordance with the following method.

< Water-Permeation Rate of Nonwoven Fabric >

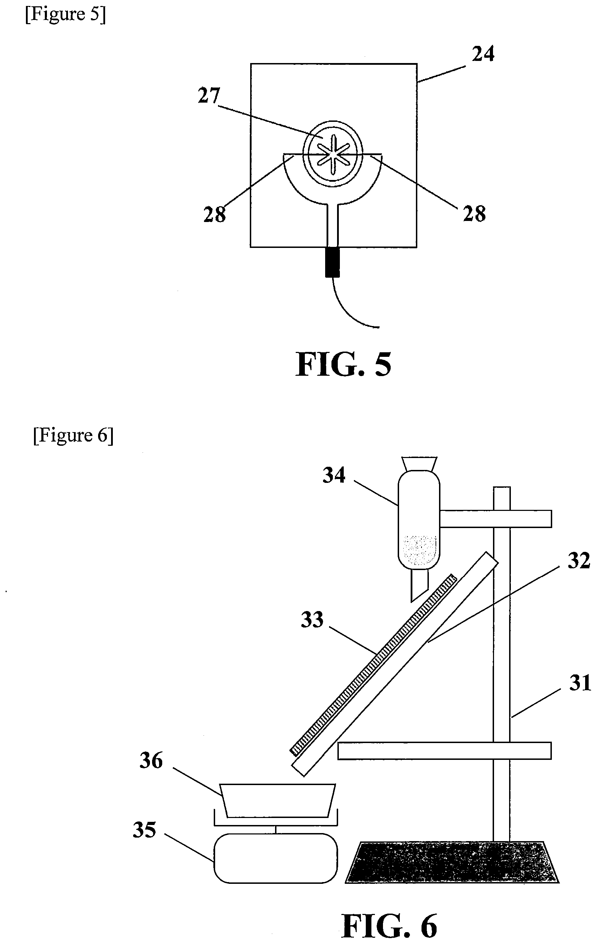

A water-permeation rate of a nonwoven fabric was measured with a strike-through time measurement instrument manufactured by INTEC CO., LTD., Model: Lister. The measurement method will be explained using Figures 4 and 5.

As shown in Figure 4, schematically, it is a mechanism in which a saline solution, an aqueous 0.9% by mass sodium chloride solution, is supplied as a test solution to a nonwoven fabric sample 24 placed on a base plate 26 through a strike-through plate 23, and the time (s) for a test solution to pass through the nonwoven fabric sample is measured. The detailed specifications are given hereinbelow.

Five sheets of filter paper (ERT FF3 Filter Paper: manufactured by Hollingsworth & Vose Company Ltd.) having dimensions of 100 mm $\times$ 100 mm were placed on a Plexiglas base plate 26 made of acrylic resin having dimensions of 125 mm $\times$ 125 mm to form a filter paper layer 25, and a nonwoven fabric sample 24 having dimensions of (125 mm $\pm$ 1 mm) $\times$ (125 mm $\pm$ 1 mm) was placed thereon. Further, a strike-through plate 23 was placed thereon in such a manner that a central hole 27 having an inner diameter of 25 mm of the strike-through plate 23 would come immediate below a magnetic valve 22.

A container 21 was charged with 15 ml of a saline solution, and the saline solution was supplied to a nonwoven fabric sample 24 from the magnetic valve 22 through the central hole 27 of the strike-through plate 23.

In the strike-through plate 23 shown in Figure 5, an electroconductive saline solution was passed through between two electrodes 28 located at the bottom of the central hole 27, so that electric current started to flow, and the timer was started. In addition, when a nonwoven fabric sample 24 was permeated, and the saline solution finished absorbing into a filter paper layer 25, the electric current was cutoff, at which time the timer stopped. The time period (s) therebetween was measured, the procedures were repeated five times, and an average thereof was defined as a water-permeation rate of a nonwoven fabric.

< Amount of Water Absorption of Nonwoven Fabric >

A nonwoven fabric was cut into dimensions of (100 mm $\pm$ 1 mm) $\times$ (100 mm $\pm$ 1 mm), and n number of the nonwoven fabrics were overlaid until the mass of a single test piece reached 1 g or more. The mass Wa (g) of this test piece was measured, and edges of the test piece was then fastened to stainless steel wire gauze with some clips. Next, the test piece and the wire gauze were sloped in a water tank charged with pure water at 20° $\pm$ 2°C, 20 mm below the water level so as not to generate bubbles, and the test piece and the wire gauze were then allowed to stand for 60 s $\pm$ 1 s. Thereafter, the test piece and the wire gauze were taken out, and other clips of the test piece were taken off while keeping a clip at one end of the test piece, and hanged vertically for 120 s $\pm$ 3 s, to drain water, and the test piece was then taken off from the wire gauze. The mass Wb of the test piece after water absorption was measured, the amount of water absorption was obtained according to the following formula:

$$\text{Amount of Water Absorption of Test Piece (g/m}^2)$$

$$= [\text{Wb - Wa}](g)/(n \times 0.01)(\text{m}^2)$$

The procedures were repeated five times, and an average thereof was defined as an amount of water absorption of a nonwoven fabric.

(Example 1 (not in accordance with the present invention))

A roller spreader (manufactured by HASHIMA CO., LTD., SINTERACE M/C) was charged at its supplying inlet with a mixture prepared by homogeneously mixing 54 parts by mass of an ethylene-vinyl acetate copolymer (EVA; melting point: 95°C) as an adhesive and 270 parts by mass of a crosslinked product of a partially neutralized polymer of acrylic acid (manufactured by Sumitomo Seika Co., Ltd., AQUAKEEP SA55SX-II) as a water-absorbent resin. On the other hand, a spunbond nonwoven fabric having a width of 15 cm (fiber: nylon, basis weight: 53 g/m², referred to as "Nonwoven Fabric C") as a water-retaining nonwoven fabric was spread over a conveyor at the bottom part of the roller spreader. Next, the spreading roller and the bottom part conveyor were operated, thereby allowing the above-mentioned mixture to evenly overlay the above-mentioned nonwoven fabric at a basis weight of 324 g/m².

The overlaid product obtained was sandwiched from the top side with an air-through nonwoven fabric (fiber: PP/PE=1/1, basis weight: 22 g/m², referred to as "Nonwoven Fabric A") as a water-permeable nonwoven fabric, and thereafter heat-fused with a laminating machine (manufactured by HASHIMA CO., LTD., straight linear fusing press HP-600LF) of which heating temperature was set at 130°C to integrate, to give a water-absorbent sheet structure. The cross section of the structure of the resulting water-absorbent sheet composition, as schematically shown, had a structure as shown in Figure 1.

The resulting water-absorbent sheet structure was cut into a given size with a water-permeable nonwoven fabric Nonwoven Fabric A on an upper side to perform various measurements and evaluations described later. The results are shown in Table 2.

(Example 2 (not in accordance with the present invention))

A spunlace nonwoven fabric having a width of 15 cm (fiber: rayon/PP/PE = 8/1/1, basis weight: 39 g/m², referred to as "Nonwoven Fabric D") as a water-retaining nonwoven fabric was spread over a hot melt applicator (manufactured by HALLYS Corporation, Marshall 150) of which heating temperature was set at 150°C, and thereafter a styrene-butadiene-styrene block copolymer (SBS, softening point: 85°C) was coated as an adhesive over the nonwoven fabric at a basis weight of 20 g/m².

Next, a roller spreader (manufactured by HASHIMA CO., LTD., SINTERACE M/C) was charged at its supplying inlet with a crosslinked product of a partially neutralized polymer of acrylic acid (manufactured by Sumitomo Seika Co., Ltd., AQUAKEEP SA55SX-II) as a water-absorbent resin. On the other hand, the above-mentioned adhesive-coated Nonwoven Fabric D was spread over a conveyor at the bottom part of the roller spreader with the adhesive-coated side being on an upper side. Subsequently, the spreading roller and the bottom part conveyor were operated, thereby allowing the water-absorbent resin to evenly overlay the nonwoven fabric at a basis weight of 270 g/m².

The overlaid product obtained was sandwiched from an upper side with an air-through nonwoven fabric (fiber: PP/PE=1/1, basis weight: 25 g/m², referred to as "Nonwoven Fabric B") as a water-permeable nonwoven fabric, coated with the above-mentioned SBS as an adhesive in the same manner as above at a basis weight of 20 g/m², and thereafter heat-fused with a laminating machine (manufactured by HASHIMA CO., LTD., straight linear fusing press HP-600LF) of which heating temperature was set at 100°C to integrate, to give a water-absorbent sheet structure. The cross section of the structure of the resulting water-absorbent sheet composition, as schematically shown, had a structure as shown in Figure 2.

The resulting water-absorbent sheet structure was cut into a given size with a water-permeable nonwoven fabric Nonwoven Fabric B on an upper side to perform various measurements and evaluations described later. The results are shown in Table 2.

(Example 3 (not in accordance with the present invention))

The same procedures as in Example 1 were carried out except that Nonwoven Fabric B was used as a water-permeable nonwoven fabric, and that a homogeneous mixture of 120 parts by mass of the adhesive and 600 parts by mass of the water-absorbent resin was dispersed at a basis weight of 720 g/m², to give a water-absorbent sheet structure. The resulting water-absorbent sheet structure was cut into a given size with a water-permeable nonwoven fabric Nonwoven Fabric B on an upper side to perform various measurements and evaluations described later. The results are shown in Table 2.

(Example 4)

The above-mentioned Nonwoven Fabric D having a width of 15 cm as a water-retaining nonwoven fabric was spread over a hot melt applicator (manufactured by HALLYS Corporation, Marshall 150) of which heating temperature was set at 150°C, and thereafter a styrene-butadiene-styrene block copolymer (SBS, softening point: 85°C) was coated as an adhesive over the nonwoven fabric at a basis weight of 11 $g/m^2$.

Next, a roller spreader (manufactured by HASHIMA CO., LTD., SINTERACE M/C) was charged at its supplying inlet with a crosslinked product of a partially neutralized polymer of acrylic acid (manufactured by Sumitomo Seika Co., Ltd., AQUAKEEP SA55SX-II) as a water-absorbent resin. On the other hand, the above-mentioned adhesive-coated Nonwoven Fabric D was spread over a conveyor at the bottom part of the roller spreader with the adhesive-coated side being on an upper side. Subsequently, the spreading roller and the bottom part conveyor were operated, thereby allowing the water-absorbent resin to evenly overlay the nonwoven fabric at a basis weight of 150 $g/m^2$.

The overlaid product obtained was sandwiched from an upper side with the above-mentioned Nonwoven Fabric A as a breathable fractionating layer, coated with the above-mentioned SBS as an adhesive in the same manner as above at a basis weight of 11 $g/m^2$, and thereafter heat-fused with a laminating machine (manufactured by HASHIMA CO., LTD., straight linear fusing press HP-600LF) of which heating temperature was set at 100°C to integrate, to give an intermediate product of a water-absorbent sheet structure.

In the same manner as above, Nonwoven Fabric A of the intermediate product of a water-absorbent sheet structure spread over the hot melt applicator of which heating temperature was set at 150°C, was coated with the above-mentioned SBS as an adhesive at a basis weight of 9 $g/m^2$.

Next, the roller spreader was charged at its supplying inlet with the water-absorbent resin. On the other hand, the intermediate product of a water-absorbent sheet structure was spread over a conveyor at the bottom side of the spreader, with an adhesive-coated side on an upper side. Subsequently, the spreading roller and the bottom side conveyor were operated, thereby allowing the water-absorbent resin to evenly overlay over the above-mentioned intermediate product of a water-absorbent sheet structure at a basis weight of 120 $g/m^2$.

The overlaid product obtained was sandwiched from an upper side with Nonwoven Fabric A as a water-permeable nonwoven fabric, coated with the above-mentioned SBS in the same manner as above at a basis weight of 9 $g/m^2$, and thereafter heat-fused with a laminating machine (manufactured by HASHIMA CO., LTD., straight linear fusing press HP-600LF) of which heating temperature was set at 100°C to integrate, to give a water-absorbent sheet structure. The cross section of the structure of the resulting water-absorbent sheet composition, as schematically shown, had a structure as shown in Figure 3.

The resulting water-absorbent sheet structure was cut into a given size with a water-permeable nonwoven fabric Nonwoven Fabric A on an upper side to perform various measurements and evaluations described later. The results are shown in Table 2.

(Example 5)

The above-mentioned Nonwoven Fabric D having a width of 15 cm as a water-retaining nonwoven fabric was spread over a hot melt applicator (manufactured by HALLYS Corporation, Marshall 150) of which heating temperature was set at 150°C, and thereafter a styrene-butadiene-styrene block copolymer (SBS, softening point: 85°C) was coated as an adhesive over the nonwoven fabric at a basis weight of 6 $g/m^2$.

Next, a roller spreader (manufactured by HASHIMA CO., LTD., SINTERACE M/C) was charged at its supplying inlet with a crosslinked product of a partially neutralized polymer of acrylic acid (manufactured by Sumitomo Seika Co., Ltd., AQUAKEEP SA55SX-II) as a water-absorbent resin. On the other hand, the above-mentioned adhesive-coated Nonwoven Fabric D was spread over a conveyor at the bottom part of the roller spreader with the adhesive-coated side being on an upper side. Subsequently, the spreading roller and the bottom part conveyor were operated, thereby allowing the water-absorbent resin to evenly overlay the nonwoven fabric at a basis weight of 50 $g/m^2$.

The overlaid product obtained was sandwiched from an upper side with a spunbond-meltblown-spunbond (SMS) nonwoven fabric (fiber: PP, basis weight: 13 $g/m^2$, referred to as "Nonwoven Fabric E") as a breathable fractionating layer, coated with the above-mentioned SBS as an adhesive in the same manner as above at a basis weight of 6 $g/m^2$, and thereafter heat-fused with a laminating machine (manufactured by HASHIMA CO., LTD., straight linear fusing press HP-600LF) of which heating temperature was set at 100°C to integrate, to give an intermediate product of a water-absorbent sheet structure.

In the same manner as above, Nonwoven Fabric E of the intermediate product of a water-absorbent sheet structure spread over the hot melt applicator of which heating temperature was set at 150°C, was coated with the above-mentioned SBS as an adhesive at a basis weight of 25 $g/m^2$.

Next, the roller spreader was charged at its supplying inlet with the water-absorbent resin. On the other hand, the intermediate product of a water-absorbent sheet structure was spread over a conveyor at the bottom side of the spreader, with an adhesive-coated side on an upper side. Subsequently, the spreading roller and the bottom side conveyor were operated, thereby allowing the water-absorbent resin to evenly overlay over the above-mentioned intermediate product of a water-absorbent sheet structure at a basis weight of 350 $g/m^2$.

The overlaid product obtained was sandwiched from an upper side with Nonwoven Fabric B as a water-permeable nonwoven fabric, coated with the above-mentioned SBS in the same manner as above at a basis weight of 25 g/m$^2$, and thereafter heat-fused with a laminating machine (manufactured by HASHIMA CO., LTD., straight linear fusing press HP-600LF) of which heating temperature was set at 100°C to integrate, to give a water-absorbent sheet structure. The cross section of the structure of the resulting water-absorbent sheet composition, as schematically shown, had a structure as shown in Figure 3.

The resulting water-absorbent sheet structure was cut into a given size with a water-permeable nonwoven fabric Nonwoven Fabric B on an upper side to perform various measurements and evaluations described later. The results are shown in Table 2.

(Comparative Example 1)

The same procedures as in Example 2 were carried out except that Nonwoven Fabric D was used in place of the water-permeable nonwoven fabric in Example 2, i.e. Nonwoven Fabric B, and that Nonwoven Fabric B was used in place of the water-retaining nonwoven fabric, i.e. Nonwoven Fabric D, to give a water-absorbent sheet structure. The water-absorbent sheet structure obtained was cut into a given size, Nonwoven Fabric D being positioned on an upper side, to perform various measurements and evaluations described later. The results are shown in Table 2.

(Comparative Example 2)

The same procedures as in Example 2 were carried out except that an air-through nonwoven fabric (fibers: PP/PE = 1/1, basis weight: 19 g/m$^2$; referred to as "Nonwoven Fabric F") was used in place of the water-permeable nonwoven fabric in Example 2, i.e. Nonwoven Fabric B, to give a water-absorbent sheet structure. The water-absorbent sheet structure obtained was cut into a given size, Nonwoven Fabric F being positioned on an upper side, to perform various measurements and evaluations described later. The results are shown in Table 2.

(Comparative Example 3)

The same procedures as in Example 2 were carried out except that Nonwoven Fabric A was used in place of the water-permeable nonwoven fabric in Example 2, i.e. Nonwoven Fabric B, and that Nonwoven Fabric F was used in place of the water-retaining nonwoven fabric, i.e. Nonwoven Fabric D, to give a water-absorbent sheet structure. The water-absorbent sheet structure obtained was cut into a given size, Nonwoven Fabric A being positioned on an upper side, to perform various measurements and evaluations described later. The results are shown in Table 2.

(Comparative Example 4)

The same procedures as in Example 4 were carried out except that Nonwoven Fabric E was used in place of the water-permeable nonwoven fabric in Example 4, i.e. Nonwoven Fabric A, to give a water-absorbent sheet structure. The water-absorbent sheet structure obtained was cut into a given size, Nonwoven Fabric E being positioned on an upper side, to perform various measurements and evaluations described later. The results are shown in Table 2.

(Comparative Example 5)

The same procedures as in Example 4 were carried out except that Nonwoven Fabric F was used in place of the water-retaining nonwoven fabric in Example 4, i.e. Nonwoven Fabric D, to give a water-absorbent sheet structure. The water-absorbent sheet structure obtained was cut into a given size, Nonwoven Fabric A being positioned on an upper side, to perform various measurements and evaluations described later. The results are shown in Table 2.

< Measurement of Thickness of Water-Absorbent Sheet Structure >

The thickness of the water-absorbent sheet structure obtained was measured with a thickness measurement instrument manufactured by Kabushiki Kaisha Ozaki Seisakusho, under the model number of J-B. As to the three measurement sites, namely the left end, the center, and the right end, were taken arbitrarily along a longitudinal direction. For example, in a case of a sheet structure having dimensions of 7.5 cm $\times$ 20 cm, the left end was set at a site 5 cm away from the left side, the center was set at a site 10 cm away therefrom, and the right end was set at a site 15 cm away therefrom. As the width direction, a central part was measured.

The measurement value for thickness was obtained by measuring three times at each site, and an average for each site was obtained. Further, the values at the left end, the center, and the right end were averaged, which was defined as a thickness of an overall water-absorbent sheet structure.

< Evaluations of Liquid Permeation Rate and Amount of Liquid Re-wet of Water-Absorbent Sheet Structure >

A water-absorbent sheet structure, which was cut into rectangular strips having dimensions of 7.5 cm × 20 cm in a manner that a longitudinal direction thereof is to be in a length direction (machine feeding direction) of the nonwoven fabric, was used as a sample.

A saline solution was colored with a small amount of Blue No. 1, to prepare a test solution.

A polyethylene air-through style porous liquid-permeable sheet having the same dimensions as the sample (7.5 cm × 20 cm) and a basis weight of 22 g/m$^2$ was placed on an upper side of a sample (water-absorbent sheet structure). In addition, underneath the sample was placed a polyethylene liquid-impermeable sheet having the same dimensions and basis weight as the sheet, to prepare a simple absorbent article. An acrylic plate having a bottom area of 16.8 cm$^2$ including a cylindrical cylinder located at the center thereof having an inner diameter of 1.9 cm and a height of 15 cm was placed near the central section of this absorbent article. Further, loads were placed on the acrylic plate, to have a state in which loads of a total weight of 780 g were applied to the sample. A 40 mL test solution was supplied to the cylinder at one time. At the same time, a time period until the test solution was completely permeated into the absorbent article was measured with a stopwatch, referred to as a first liquid permeation rate (s). Next, the same procedures were carried out 3 minutes after the termination of absorption of the test solution, to measure a second liquid permeation rate (s). Further, the same procedures were carried out 3 minutes after the termination of absorption of the second test solution, to measure a third liquid permeation rate (s). A total of the number of seconds for the first to third liquid permeation rates is referred to as a total liquid permeation rate.

After 10 minutes from the termination of absorption of the third test solution, the acrylic plate was removed, filter papers (10 sheets) having a diameter of 55 mm, of which mass (Wc (g), about 3 g) was previously measured, were stacked near the liquid supplying position of the absorbent article, and a 700 g weight of which diameter was 50 mm was placed thereon. After applying the load for 1 minute, the mass (Wd (g)) of the filter papers was measured, and an increased mass was defined as the amount of liquid re-wet (g) as follows.

$$\text{Amount of Liquid Re-wet (g)} = \text{Wd} - \text{Wc (g)}$$

< Evaluation of Slope Leakage Amount >

A slope leakage amount was measured using an apparatus shown in Figure 6.

Schematically, a commercially available stand 31 for experimental facilities was used to slope an acrylic plate 32 and fixed, the above-mentioned test solution was then supplied to an absorbent article 33 placed on the plate from a dropping funnel 34 positioned vertically above the absorbent article, and a leakage amount was measured with a balance 35. The detailed specifications are given hereinbelow.

An acrylic plate 32 has a length in the direction of the slope plane of 45 cm, and fixed so that an angle formed with a stand 31 against the horizontal is 45° ± 2°. The acrylic plate 32 had a width of 100 cm and a thickness of 1 cm, and plural absorbent articles 33 could be concurrently measured. The acrylic plate 32 had a smooth surface, so that a liquid was not detained or absorbed to the plate.

A dropping funnel 34 was fixed at a position vertically above the sloped acrylic plate 32 using the stand 31. The dropping funnel 34 had a volume of 100 mL, and an inner diameter of a tip end portion of about 4 mm, and an aperture of the cock was adjusted so that a liquid was supplied at a rate of 10 mL/s.

A balance 35 on which a tray 36 was placed was set at a lower side of the acrylic plate 32, and all the test solutions flowing down the plate were received as leakage, and its mass was recorded to the accuracy of 0.1 g.

A slope leakage test using an apparatus as described above was carried out in accordance with the following procedures. The mass of a water-absorbent sheet structure cut into a rectangular strip having dimensions of width 7.5 cm × length 20 cm in a manner that the longitudinal direction is a length direction (machine feeding direction) of the nonwoven fabric was measured, and an air through-style polyethylene liquid-permeable nonwoven fabric (basis weight: 22 g/m$^2$) of the same size was attached from an upper side thereof, and further a polyethylene liquid-impermeable sheet having the same basis weight of the same size was attached from a lower side thereof to prepare a simple absorbent article 33. The simple absorbent article 33 was adhered on the acrylic plate 32 (in order not to stop leakage intentionally, the bottom end of the absorbent article 33 was not adhered to the acrylic plate 32).

Marking was put on the absorbent article 33 at a position 2 cm away in a downward direction from a top end thereof, and a supplying port for the dropping funnel 34 was fixed so that the port was positioned at a distance 10 mm ± 1 mm vertically above the marking.

A balance 35 was turned on, and tared so that the indication was zero, and thereafter 20 mL of the above-mentioned test solution was supplied at one time to the dropping funnel 34. An amount of liquid poured into a tray 36 after the test solution was allowed to flow over a sloped acrylic plate 32 without being absorbed into an absorbent article 33 was measured, and this amount of liquid was defined as a first leakage amount (g).

Second and third test solutions were supplied in 10-minute intervals from the beginning of the first supply, and second and third leakage amounts (g) were measured. A total of the first to third slope leakage amounts (g) was defined as a total leakage amount.

Various properties of the nonwoven fabrics used in Examples and Comparative Examples are shown in Table 1, and the constitution and the evaluation of properties of the water-absorbent sheet structure are shown in Table 2.

[Table 1]

Table 1

| Abbreviations | Fibers | Basis Weight (g/m$^2$) | Water-Permeation Rate (s) | Amount of Water Absorption (g/m$^2$) |
|---|---|---|---|---|
| Nonwoven Fabric A | PP/PE=1/1 | 22 | 8.5 | 318 |
| Nonwoven Fabric B | PP/PE=1/1 | 25 | 7.5 | 193 |
| Nonwoven Fabric C | Nylon | 53 | 14.2 | 300 |
| Nonwoven Fabric D | Rayon/PP/PE=8/1/1 | 39 | 11.1 | 385 |
| Nonwoven Fabric E | PP | 13 | 12.0 | 105 |
| Nonwoven Fabric F | PP/PE=1/1 | 19 | 10.5 | 228 |

[Table 2]

Table 2

| | Nonwoven Fabric | | Liquid-Permeable Fractionating Layer | Water-Absorbent Resin (g/m²) | | Adhesive (g/m²) | | Thickness (mm) | Water-Absorbent Sheet Structure | | | | | | | | |
|---|---|---|---|---|---|---|---|---|---|---|---|---|---|---|---|---|---|
| | Upper Side | Lower Side | | Primary Absorbent Layer | Secondary Absorbent Layer | Primary Absorbent Layer | Secondary Absorbent Layer | | Liquid Permeation Rate (s) | | | | Amount of Liquid Re-wet (g) | Slope Leakage Amount (g) | | | |
| | | | | | | | | | First | Second | Third | Total | | First | Second | Third | Total |
| Ex. 1 | A | C | - | 270 | | 54 | | 1.4 | 118 | 161 | 207 | 486 | 6.7 | 0.7 | 0 | 0 | 0.7 |
| Ex. 2 | B | D | - | 270 | | 40 | | 1.5 | 110 | 146 | 197 | 453 | 6.2 | 0.5 | 0 | 0 | 0.5 |
| Ex. 3 | B | C | - | 600 | | 120 | | 2.0 | 135 | 180 | 225 | 540 | 4.3 | 0.3 | 0 | 0 | 0.3 |
| Ex. 4 | A | D | Nonwoven Fabric A | 120 | 150 | 18 | 22 | 1.8 | 82 | 182 | 239 | 50s | 7.3 | 0 | 0 | 0 | 0 |
| Ex. 5 | B | D | Nonwoven Fabric E | 350 | 50 | 50 | 12 | 2.0 | 123 | 184 | 227 | 534 | 5.5 | 0 | 0 | 0 | 0 |
| Comp. Ex. 1 | D | B | - | 270 | | 40 | | 1.5 | 166 | 217 | 274 | 657 | 14.3 | 1.9 | 0.5 | 0.1 | 2.5 |
| Comp. Ex. 2 | F | D | - | 270 | | 40 | | 1.4 | 148 | 201 | 262 | 611 | 12.9 | 0.7 | 0.2 | 0 | 0.9 |
| Comp. Ex. 3 | A | F | - | 270 | | 40 | | 1.5 | 117 | 159 | 203 | 479 | 9.8 | 1.4 | 0.2 | 0 | 1.6 |
| Comp. Ex. 4 | E | D | Nonwoven Fabric A | 120 | 150 | 18 | 22 | 1.7 | 146 | 253 | 309 | 708 | 11.3 | 0 | 0 | 0 | 0 |
| Comp. Ex. 5 | A | F | Nonwoven Fabric A | 120 | 150 | 18 | 22 | 1.9 | 85 | 187 | 247 | 519 | 10.4 | 1 | 0.2 | 0 | 1.2 |

13

It can be seen from the above results that the water-absorbent sheet structures of Examples 1 to 5 have fast liquid permeation rates, small amounts of liquid re-wet, and small slope leakage amounts, as compared to those of Comparative Examples 1 to 5.

On the other hand, when the comparative examples are studied, when a water permeation rate of a nonwoven fabric on an upper side, i.e. a side from which a liquid was supplied exceeds 10 s (Comparative Examples 1, 2 and 4), the liquid permeation rate is likely to be slow, and the amount of liquid re-wet is likely to be large. Also, when an amount of water absorption of a nonwoven fabric on a lower side, i.e. a side opposite from which a liquid is supplied, is less than 250 g/m$^2$ (Comparative Examples 1, 3 and 5), the slope leakage amount would be large.

INDUSTRIAL APPLICABILITY

The water-absorbent sheet structure of the present invention can be used for absorbent articles in the fields of hygienic materials, agricultural fields, fields of construction materials, and the like, and especially the water-absorbent sheet structure can be suitably used in hygienic material fields such as incontinence pads.

EXPLANATION OF NUMERICAL SYMBOLS

10  water-absorbent sheet structure
11  adhesive
12  water-absorbent resin
13  absorbent layer
14  water-permeable nonwoven fabric
15  water-retaining nonwoven fabric
16  adhesive
17  breathable fractionating layer
18  primary absorbent layer
19  secondary absorbent layer
21  container
22  magnetic valve
23  strike-through plate
24  nonwoven fabric sample
25  filter paper layer
26  base plate
27  central hole
28  electrode
31  stand
32  acrylic plate
33  absorbent article
34  dropping funnel
35  balance
36  tray

**Claims**

1. A water-absorbent sheet structure (10) comprising a structure in which an absorbent layer (18, 19) comprising a water-absorbent resin (12) and an adhesive (16) is sandwiched with nonwoven fabrics (14, 15) from an upper side and a lower side of the absorbent layer (18, 19), the water-absorbent sheet structure (10) comprising:

   (1) an upper nonwoven fabric (14) which is a water-permeable nonwoven fabric having a water permeation rate of 10 s or less; and
   (2) a lower nonwoven fabric (15) which is a water-retaining nonwoven fabric having an amount of water absorption of 250 g/m$^2$ or more,

   wherein the absorbent layer (18, 19) is fractionated into a primary absorbent layer (18) and a secondary absorbent layer (19), with a breathable fractionating layer (17).

2. The water-absorbent sheet structure (10) of claim 1, wherein the breathable fractionating layer (17) is a layer through which the water-absorbent resin (12) does not substantially pass.

3. The water-absorbent sheet structure (10) of claim 1 or 2, wherein the breathable fractionating layer (17) is made of PE or PP fibers; porous films; sanitary papers; tissue paper; cellulose-containing synthetic fiber nonwoven fabrics; airlaid nonwoven fabrics made of pulp/PE/PP, or nonwoven fabrics made of synthetic fibers; rayon fibers, polyolefin fibers, and polyester fibers.

4. The water-absorbent sheet structure (10) of claim 1, 2 or 3, wherein a mass ratio of the primary absorbent layer (18) to the secondary absorbent layer (19) is from 98/2 to 20/80, preferably from 95/5 to 25/75 and most preferably from 90/10 to 30/70.

5. The water-absorbent sheet structure (10) of any of claims 1 to 4, wherein the water absorbent sheet structure (10) is 4mm thick or less in a dry state.

6. The water-absorbent sheet structure (10) according to any of claims 1-5, wherein the water-permeable nonwoven fabric is a nonwoven fabric made of polyolefin fibers or polyester fibers.

7. The water-absorbent sheet structure (10) according to any of claims 1-6, wherein the water-retaining nonwoven fabric is a spunbond nonwoven fabric made of polyamide fibers, or spunlace nonwoven fabric made of rayon fabrics as a main component.

8. The water-absorbent sheet structure (10) according to any one of claims 1 to 7, wherein the adhesive (16) is at least one member selected from the group consisting of ethylene-vinyl acetate copolymer adhesives, styrene-based elastomer adhesives, polyolefin-based adhesives, and polyester-based adhesives.

9. An absorbent article (33) comprising the water-absorbent sheet structure (10) as defined in any one of claims 1 to 8, sandwiched between a liquid-permeable sheet and a liquid-impermeable sheet.

**Patentansprüche**

1. Wasserabsorbierende Plattenstruktur (10), umfassend eine Struktur, in der eine Absorptionsschicht (18, 19), die ein wasserabsorbierendes Harz (12) und einen Klebstoff (16) umfasst, mit Vliesstoffen (14, 15) von einer oberen Seite und einer unteren Seite der Absorptionsschicht (18, 19) eingeschlossen ist, wobei die wasserabsorbierende Plattentruktur (10) Folgendes umfasst:

(1) einen oberen Vliesstoff (14), der ein wasserdurchlässiger Vliesstoff mit einer Wasserdurchlässigkeitsrate von 10 s oder weniger ist; und
(2) einen unteren Vliesstoff (15), der ein wasserhaltender Vliesstoff mit einer Wasserabsorptionsmenge von 250 g/m$^2$ oder mehr ist,

wobei die Absorptionsschicht (18, 19) in eine primäre Absorptionsschicht (18) und eine sekundäre Absorptionsschicht (19) mit einer atmungsaktiven Fraktionierungsschicht (17) fraktioniert wird.

2. Wasserabsorbierende Plattenstruktur (10) nach Anspruch 1, wobei die atmungsaktive Fraktionierungsschicht (17) eine Schicht ist, durch die das wasserabsorbierende Harz (12) im Wesentlichen nicht verläuft.

3. Wasserabsorbierende Plattenstruktur (10) nach Anspruch 1 oder 2, wobei die atmungsaktive Fraktionierungsschicht (17) aus PE- oder PP-Fasern; porösen Folien; Hygienepapier; Tissuepapier; zellstoffhaltigen Kunstfaser-Vliesstoffen; aus Zellstoff/PE/PP hergestellten Airlaid-Vliesstoffen oder aus Kunstfasern hergestellten Vliesstoffen; Rayonfasern, Polyolefinfasern und Polyesterfasern hergestellt ist.

4. Wasserabsorbierende Plattenstruktur (10) nach Anspruch 1, 2 oder 3, wobei ein Massenverhältnis der primären Absorptionsschicht (18) zu der sekundären Absorptionsschicht (19) von 98/2 bis 20/80, bevorzugt von 95/5 bis 25/75 und am bevorzugtesten von 90/10 bis 30/70 ist.

5. Wasserabsorbierende Plattenstruktur (10) nach einem der Ansprüche 1 bis 4, wobei die wasserabsorbierende Plattenstruktur (10) 4 mm dick oder weniger in einem trockenen Zustand ist.

**6.** Wasserabsorbierende Plattenstruktur (10) nach einem der Ansprüche 1 bis 5, wobei der wasserdurchlässige Vliesstoff ein Vliesstoff ist, der aus Polyolefinfasern oder Polyesterfasern hergestellt ist.

**7.** Wasserabsorbierende Plattenstruktur (10) nach einem der Ansprüche 1 bis 6, wobei der wasserhaltende Vliesstoff ein aus Polyamidfasern hergestellter Spinnvliesstoff oder ein aus Rayonstoffen als Hauptbestandteil hergestellter Spunlace-Vliesstoff ist.

**8.** Wasserabsorbierende Plattenstruktur (10) nach einem der Ansprüche 1 bis 7, wobei der Klebstoff (16) mindestens ein Element ist, das ausgewählt ist aus der Gruppe bestehend aus Ethylenvinylacetat-Copolymerklebstoffen, Styrol-basierten Elastomerklebstoffen, Polyolefin-basierten Klebstoffen und Polyester-basierten Klebstoffen.

**9.** Absorptionsartikel (33), umfassend die wasserabsorbierende Plattenstruktur (10) nach einem der Ansprüche 1 bis 8, die zwischen einer flüssigkeitsdurchlässigen Platte und einer flüssigkeitsundurchlässigen Platte eingeschlossen ist.

**Revendications**

**1.** Structure en feuille absorbant l'eau (10) comprenant une structure dans laquelle une couche absorbante (18, 19) comprenant une résine absorbant l'eau (12) et un adhésif (16) est prise en sandwich entre des toiles non tissées (14, 15) depuis un côté supérieur et un côté inférieur de la couche absorbante (18, 19), la structure en feuille absorbant l'eau (10) comprenant :

(1) une toile non tissée supérieure (14) qui est une toile non tissée perméable à l'eau présentant un taux de perméation d'eau de 10s ou moins ; et
(2) une toile non tissée inférieure (15) qui est une toile non tissée rétentrice d'eau présentant une quantité d'absorption d'eau de 250 g/m$^2$ ou plus,

la couche absorbante (18, 19) étant fractionnée en une couche absorbante primaire (18) et une couche absorbante secondaire (19), avec une couche de fractionnement perméable (17).

**2.** Structure en feuille absorbant l'eau (10) selon la revendication 1, dans laquelle la couche de fractionnement perméable (17) est une couche à travers laquelle la résine absorbant l'eau (12) ne passe pas sensiblement.

**3.** Structure en feuille absorbant l'eau (10) selon la revendication 1 ou 2, dans laquelle la couche de fractionnement perméable (17) est constituée de fibres de PE ou PP ; de films poreux ; de papiers hygiéniques ; de papier absorbant ; de toiles non tissées de fibre synthétique contenant la cellulose ; de toiles non tissées aéroliques constituées de pâte à papier/PE/PP, ou de toiles non tissées constituées de fibres synthétiques ; de fibres de rayonne, de fibres de polyoléfine et de fibres de polyester.

**4.** Structure en feuille absorbant l'eau (10) selon la revendication 1, 2 ou 3, dans laquelle un rapport de masse de la couche absorbante primaire (18) à la couche absorbante secondaire (19) va de 98/2 à 20/80, de préférence de 95/5 à 25/75 et idéalement de 90/10 à 30/70.

**5.** Structure en feuille absorbant l'eau (10) selon l'une quelconque des revendications 1 à 4, la structure en feuille absorbant l'eau (10) étant de 4 mm d'épaisseur ou moins dans un état sec.

**6.** Structure en feuille absorbant l'eau (10) selon l'une quelconque des revendications 1 à 5, dans laquelle la toile non tissée perméable à l'eau est une toile non tissée constituée de fibres de polyoléfine ou de fibres de polyester.

**7.** Structure en feuille absorbant l'eau (10) selon l'une quelconque des revendications 1 à 6, dans laquelle la toile non tissée rétentrice d'eau est une toile non tissée filée-liée constituée de fibres de polyamide, ou une toile non tissée lacée par filage constituée de tissus en rayonne comme composant principal.

**8.** Structure en feuille absorbant l'eau (10) selon l'une quelconque des revendications 1 à 7, dans laquelle l'adhésif (16) est au moins un élément choisi dans le groupe constitué par des adhésifs de copolymère d'acétate de vinyle-éthylène, des adhésifs en élastomère à base de styrène, des adhésifs à base de polyoléfine et des adhésifs à base de polyester.

9. Article absorbant (33) comprenant la structure en feuille absorbant l'eau (10) telle que définie dans l'une quelconque des revendications 1 à 8, prise en sandwich entre une feuille perméable au liquide et une feuille imperméable au liquide.

[Figure 1]

**FIG. 1**

[Figure 2]

**FIG. 2**

[Figure 3]

**FIG. 3**

[Figure 4]

**FIG. 4**

[Figure 5]

**FIG. 5**

[Figure 6]

**FIG. 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2383115 A1
- WO 2010004894 A
- WO 2010004895 A